# EUROPEAN PATENT SPECIFICATION

(11) **EP 2 362 763 B1**
(45) Date of publication and mention of the grant of the patent: **14.11.2012**
(21) Application number: 09741529.3
(22) Date of filing: 14.10.2009
(51) Int. Cl.: A61F 9/007, B25B 13/48

(54) **SYSTEMS AND METHODS FOR TIGHTENING OPHTHALMIC SURGICAL NEEDLES**
SYSTEME UND VERFAHREN ZUM FESTZIEHEN VON NADELN FÜR AUGENOPERATIONEN
SYSTEMES ET PROCEDES D'AJUSTEMENT D'AIGUILLES POUR CHIRURGIE OPHTALMIQUE

(30) Priority: 14.10.2008 US 250602
(43) Date of publication of application: 07.09.2011
(73) Proprietor: Bausch & Lomb Incorporated, Rochester, NY 14604-2701 (US)
(72) Inventor: BURGETT, Seth, D., Glen Carbon IL 62034 (US)
(74) Representative: Vossius & Partner
(86) International application number: PCT/US2009/060607
(87) International publication number: WO 2010/045300

(56) References cited:
- WO-A1-90/04362
- WO-A2-00/74615
- US-A1- 2007 239 102

## Description

### Background

### 1. Field

The present disclosure is directed to systems and methods for tightening ophthalmic surgical needles onto surgical handpieces.

### 2. Description of the Related Art

The statements in this section merely provide background information related to the present disclosure and may not constitute prior art.

In eye surgery, particularly phacoemulsification surgery, the use of ophthalmic surgical needles is commonly required in cataract surgeries. Ophthalmic surgical needles transfer ultrasonic energy through a surgical handpiece to a cataract within a patient's eye. It is desired to have proper contact between the ophthalmic surgical needle and the surgical handpiece to provide efficient transmission of ultrasonic energy and reliable connection of the ophthalmic surgical needle. Proper connection between the ophthalmic surgical needle and the surgical handpiece is generally achieved by threading the ophthalmic surgical needle onto the surgical handpiece.

Presently, a user threads an ophthalmic surgical needle onto the surgical handpiece by hand, and then uses a disposable wrench, provided with each ophthalmic surgical needle, to tighten the ophthalmic surgical needle. The disposable wrench is a one-use tool and requires the user to use two hands while tightening the ophthalmic surgical needle onto the surgical handpiece.

### Brief Description of the Drawings

The drawings described herein are for illustration purposes only and are not intended to limit the scope of the present disclosure in any way.
FIG. 1 is perspective view of an ophthalmic surgery system including a power wrench assembly according to the present disclosure; and
FIG. 2 is a partial elevation view of a handpiece and ophthalmic surgical needle for use with ophthalmic surgery systems according to the present disclosure.

### Detailed Description of the Preferred Embodiment

The following description is merely exemplary in nature and is not intended to limit the present disclosure, application, or uses.

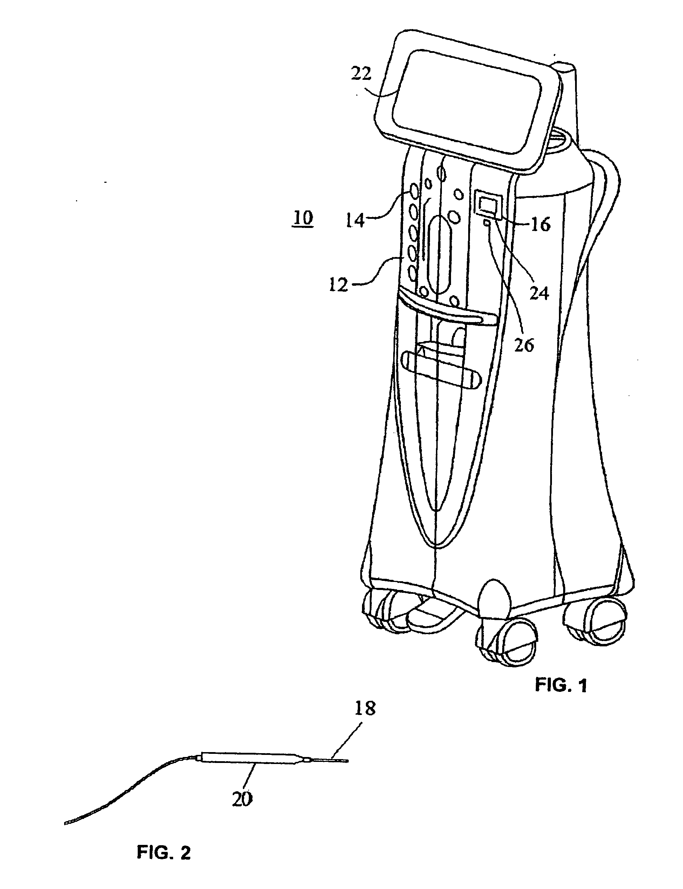

According to one embodiment of the present disclosure, an ophthalmic surgery system 10 is illustrated in FIG. 1. The ophthalmic surgery system 10 includes a pump 12 for generating fluid flow during ophthalmic surgery and a handpiece interface 14 for providing control of a surgical handpiece. The ophthalmic surgery system 10 also includes a power wrench assembly 16 for interfacing an ophthalmic surgical needle at least partially threaded onto the surgical handpiece. One exemplary embodiment of an ophthalmic surgical needle 18 and a surgical handpiece 20 are illustrated in FIG. 2. The ophthalmic surgical needles and surgical handpieces as described herein are generally used for phacoemulsification surgical procedures.

The power wrench assembly 16 is configured to rotate the ophthalmic surgical needle relative to the surgical handpiece until torque applied to the ophthalmic surgical needle is within a desired torque range. In this manner, tightening the ophthalmic surgical needle is automated (i.e., not by a user) thereby reducing potential human errors in achieving the desired torque range and enhancing connection of the ophthalmic surgical needle thereby improving the tuning process of ophthalmic surgery system 10.

Automatically tightening the ophthalmic surgical needle further reduces the overall time needed to prepare the surgical handpiece for surgery. Additionally, the power wrench assembly 16 also permits a user, such as a technician, clinician, nurse, etc., to conveniently tighten an ophthalmic surgical needle onto a surgical handpiece with only one hand. Also, additional tools for use with an ophthalmic surgical needle, e.g., the disposable tools, may be eliminated thereby reducing the cost of each ophthalmic surgical procedure.

In use, a user partially threads an ophthalmic surgical needle onto a surgical handpiece. The surgical handpiece is then positioned proximal to the power wrench assembly 16 with the ophthalmic surgical needle extending into an opening defined by the power wrench assembly 16. In this manner, ophthalmic surgery system 10 eliminates risk of potential puncture injuries to the user during tightening of the ophthalmic surgical needle. With the surgical handpiece positioned proximal to the power wrench assembly 16, the user applies pressure to the surgical handpiece thereby biasing the ophthalmic surgical needle and/or surgical handpiece against the power wrench assembly 16. When the power wrench assembly 16 detects the biasing, the power wrench assembly 16 rotates the ophthalmic surgical needle relative to the surgical handpiece. The power wrench assembly 16 continues to rotate the ophthalmic surgical needle, increasing torque applied to the ophthalmic surgical needle as necessary. When the torque applied to the ophthalmic surgical needle is within a desired range, the power wrench assembly 16 discontinues rotating the ophthalmic surgical needle automatically.

The desired range may be provided by a manufacturer of one or more of the ophthalmic surgical needle, the surgical handpiece, and ophthalmic surgical system. The desired torque range is generally any range sufficient to provide a proper connection between an ophthalmic surgical needle and a handle piece. Further, the desired torque range may also be provided by a user via a user-interface to the ophthalmic surgery system 10 or preprogrammed into the ophthalmic surgery system 10.

The ophthalmic surgery system 10 includes an indicator 22 for providing a visual and audible indication to a user indicating when the torque applied to the ophthalmic surgical needle is within the desired range. While the indicator 22 included in the ophthalmic surgery system 10 is visual and audible, it should be appreciated that various types of audio and/or visual indicators can be employed in other embodiments of the present disclosure. In this embodiment, the indicator 22 is a display associated within the surgery system for providing other information related to the power wrench assembly 16 and/or relevant to the ophthalmic surgery, such as flow information related to the pump 12 or monitoring of the patient. In still other embodiments, an indicator may be omitted, whereby a user may be prompted to remove the surgical handpiece by the power wrench assembly discontinuing rotation of the ophthalmic surgical needle.

When torque applied to the ophthalmic surgical needle is within a desired torque range as provided by the indicator 22, the user may remove the surgical handpiece with the secured ophthalmic surgical needle from the power wrench assembly 16 for use in ophthalmic surgery.

After the ophthalmic surgery, the ophthalmic surgical needle and surgical handpiece are removed from the ophthalmic surgery system 10. For a subsequent ophthalmic surgery, another surgical handpiece is coupled to the ophthalmic surgery system 10 via the handpiece interface 14. An ophthalmic surgical needle is threaded onto the surgical handpiece by the user and presented to the power wrench assembly for tightening. Before the surgical handpiece is positioned proximal to the power wrench assembly 16, a removable wrench insert 24 included in the power wrench assembly 16 is replaced. The removable wrench insert 24 is configured to contact the ophthalmic surgical needle. Contact between power wrench assembly 16 and the ophthalmic surgical needle and surgical handpiece in generally limited to the removable wrench insert 24. By replacing the removable wrench insert 24, a sterile contact is preserved between the power wrench assembly and the ophthalmic surgical needle and the surgical handpiece for the subsequent ophthalmic surgery.

In some embodiments, a removable wrench insert 24 may be reusable when sterilized, *e.g*., autoclaved, or the insert 24 may be disposable.

Referring again to FIG. 1, the ophthalmic surgery system 10 also includes a sensor 26 for monitoring when a removable wrench insert is removed. Based on an output from the sensor 26, the ophthalmic surgery system 10 may be configured to indicate when replacement of the removable wrench insert is necessary. In the embodiment illustrated in FIG. 1, replacement of the removable wrench insert is generally necessary for each patient. By indicating replacement of the removable wrench insert is necessary, the ophthalmic surgery system 10 is a convenient and reliable indicator to the user to further aid in ensuring a sterilized contact with the power wrench assembly for each patient. Specifically, the inventors hereof have recognized that in some embodiments, an ophthalmic surgery system with a power wrench assembly and a sensor monitoring a removable wrench insert may reduce the potential of reusing unsterilized instruments, *e.g*., disposable tools, for different patients.

In other embodiments, the ophthalmic surgery system 10 may be configured to indicate replacement of a removable wrench insert is necessary after each use. The particular timing of the indication may vary for different embodiments of the present disclosure. For example, an indication may be provided to a user when the user attempts to tighten a second ophthalmic surgical needle via the power wrench assembly 16 without replacing the removable wrench insert 24. The indication may be displayed via indicator 22 or a different visual and/or audible indicator included in the ophthalmic surgery system 10. For example, an LED may be included adjacent to the power wrench assembly such that the LED is within a user's field of view when using the power wrench assembly.

Although several aspects of the present disclosure have been described above with reference to phacoemulsification instruments, it should be understood that various aspects of the present disclosure are not limited to phacoemulsification instruments, and can be applied to a variety of other ophthalmic surgical procedures.

By implementing any or all of the teachings described above, a number of benefits and advantages can be attained including improved reliability, reduced down time, elimination or reduction of redundant components or systems, avoiding unnecessary or premature replacement of components or systems, and a reduction in overall system and operating costs.

## Claims

1. An ophthalmic surgery system (10) comprising:
a pump (12) for generating fluid flow during ophthalmic surgery;
a handpiece (14) interface for providing control of a surgical handpiece;
and **characterized in that** the system further comprises:
a power wrench assembly (16) for interfacing with an ophthalmic surgical needle; and
wherein the power wrench assembly (16) is configured to rotate the ophthalmic surgical needle (18) relative to the surgical handpiece (20).

2. The ophthalmic surgery system of claim 1, wherein the power wrench assembly is configured to automatically rotate the ophthalmic surgical needle when a user biases at least one of the ophthalmic surgical needle and the surgical handpiece against the power wrench assembly.

3. The ophthalmic surgery system of claim 1, further comprising an indicator (22) for providing a visual and/or audible indication to a user when the torque applied to the ophthalmic surgical needle is within the desired torque range.

4. The ophthalmic surgery system of claim 1, wherein the power wrench assembly includes a removable wrench insert (24) for contacting an ophthalmic surgical needle.

5. The ophthalmic surgery system of claim 4, wherein the removable wrench insert is autoclavable.

6. The ophthalmic surgery system of claim 1, further comprising a sensor (26) for monitoring a removable wrench Insert, wherein based on an output from the sensor, the ophthalmic surgery system is configured to Indicate to a user when replacement of the removable wrench insert is necessary.

7. The ophthalmic surgery system of claim 1, further comprising an indicator for indicating to a user when replacement of the removable wrench insert is necessary.

8. A method for tightening an ophthalmic surgical needle (18) onto a surgical handpiece (20) via an ophthalmic surgery system (10), the method comprising:
providing the system (10) with a power wrench assembly (18);
detecting at least one of the ophthalmic surgical needle (18) and the surgical handpiece (20); and
automatically rotating the ophthalmic surgical needle (18) relative to the surgical handpiece (20) until torque applied to the ophthalmic surgical needle is within a desired torque range.

9. The method of claim 8, further comprising notifying a user when the torque applied to the ophthalmic surgical needle is within the desired torque range via a visual and/or audible Indicator (22) included In the ophthalmic surgery system.

10. The method of claim 8, further comprising notifying a user when replacement of a removable wrench insert (24) is necessary.

## Patentansprüche

1. Augenoperationssystem (10), das aufweist:
eine Pumpe (12) zum Erzeugen von Fluiddurchfluss während einer Augenoperation;
eine Handstückschnittstelle (14) zur Steuerung eines Operationshandstücks;
und **dadurch gekennzeichnet, dass** das System ferner aufweist:
eine Kraftschrauberanordnung (16) zum Koppeln mit einer Augenoperationsnadel; und
wobei die Kraftschrauberanordnung (16) so konfiguriert ist, dass sie die Augenoperationsnadel (18) relativ zum Operationshandstück (20) dreht.

2. Augenoperationssystem nach Anspruch 1, wobei die Kraftschrauberanordnung so konfiguriert ist, dass sie die Augenoperationsnadel automatisch dreht, wenn ein Benutzer die Augenoperationsnadel und/oder das Operationshandstück gegen die Kraftschrauberanordnung vorspannt.

3. Augenoperationssystem nach Anspruch 1, ferner mit einem Anzeiger (22) zum Bereitstellen einer Sicht- und/oder Höranzeige für einen Benutzer, wenn das auf die Augenoperationsnadel ausgeübte Drehmoment im Solldrehmomentbereich liegt.

4. Augenoperationssystem nach Anspruch 1, wobei die Kraftschrauberanordnung einen entfernbaren Schraubereinsatz (24) zum Kontaktieren einer Augenoperationsnadel aufweist.

5. Augenoperationssystem nach Anspruch 4, wobei der entfernbare Schraubereinsatz autoklavierbar ist.

6. Augenoperationssystem nach Anspruch 1, ferner mit einem Sensor (26) zum Überwachen eines entfernbaren Schraubereinsatzes, wobei auf der Grundlage einer Ausgabe vom Sensor das Augenoperationssystem so konfiguriert ist, dass es einem Benutzer anzeigt, wenn ein Austausch des entfernbaren Schraubereinsatzes notwendig ist.

7. Augenoperationssystem nach Anspruch 1, ferner mit einem Anzeiger zum Anzeigen für einen Benutzer, wenn ein Austausch des entfernbaren Schraubereinsatzes notwendig ist.

8. Verfahren zum Festziehen einer Augenoperationsnadel (18) an einem Operationshandstück (20) über ein Augenoperationssystem (10), wobei das Verfahren aufweist:
Versehen des Systems (10) mit einer Kraftschrauberanordnung (16);
Detektieren der Augenoperationsnadel (18) und/oder des Operationshandstücks (20); und
automatisches Drehen der Augenoperationsnadel (18) relativ zum Operationshandstück (20), bis ein auf die Augenoperationsnadel ausgeübtes Drehmoment in einem Solldrehmomentbereich liegt.

9. Verfahren nach Anspruch 8, das ferner aufweist: Benachrichtigen eines Benutzers, wenn das auf die Augenoperationsnadel ausgeübte Drehmoment im Solldrehmomentbereich liegt, über einen Sicht- und/oder Höranzeiger (22), der zum Augenoperationssystem gehört.

10. Verfahren nach Anspruch 8, das ferner aufweist: Benachrichtigen eines Benutzers, wenn ein Austausch eines entfernbaren Schraubereinsatzes (24) notwendig ist.

## Revendications

1. Système de chirurgie ophtalmique (10), comprenant :
une pompe (12) pour la génération d'un écoulement de fluide pendant une chirurgie ophtalmique ;
une interface d'embout à main (14) pour fournir un contrôle d'un embout à main chirurgical ;
et **caractérisé en ce que** le système comprend également :
un ensemble boulonneuse (16) destiné à l'interfaçage avec une aiguille chirurgicale ophtalmique ;
l'ensemble boulonneuse (16) étant configuré pour faire tourner l'aiguille chirurgicale ophtalmique (18) par rapport à l'embout à main chirurgical (20).

2. Système de chirurgie ophtalmique selon la revendication 1, dans lequel l'ensemble boulonneuse est configuré pour faire tourner automatiquement l'aiguille chirurgicale ophtalmique lorsqu'un utilisateur pousse l'aiguille chirurgicale ophtalmique et/ou l'embout à main chirurgical contre l'ensemble boulonneuse.

3. Système de chirurgie ophtalmique selon la revendication 1, comprenant également un indicateur (22) destiné à fournir une indication visuelle et/ou auditive à un utilisateur lorsque le couple appliqué à l'aiguille chirurgicale ophtalmique est dans la plage de couple souhaitée.

4. Système de chirurgie ophtalmique selon la revendication 1, dans lequel l'ensemble boulonneuse comprend un insert de clé amovible (24) destiné à rentrer en contact avec une aiguille chirurgicale ophtalmique.

5. Système de chirurgie ophtalmique selon la revendication 4, dans lequel l'insert de clé amovible est autoclavable.

6. Système de chirurgie ophtalmique selon la revendication 1, comprenant également un capteur (26) pour surveiller un insert de clé amovible, le système de chirurgie ophtalmique étant configuré pour indiquer à un utilisateur lorsqu'un remplacement de l'insert de clé amovible est nécessaire sur la base d'une sortie du capteur.

7. Système de chirurgie ophtalmique selon la revendication 1, comprenant également un indicateur pour indiquer à un utilisateur lorsqu'un remplacement de l'insert de clé amovible est nécessaire.

8. Procédé de serrage d'une aiguille chirurgicale ophtalmique (18) sur un embout à main chirurgical (20) par un système de chirurgie ophtalmique (10), le procédé comprenant :
l'équipement du système (10) avec un ensemble boulonneuse (16) ;
la détection de l'aiguille chirurgicale ophtalmique (18) et/ou de l'embout à main chirurgical (20) ; et
la rotation automatique de l'aiguille chirurgicale ophtalmique (18) par rapport à l'embout à main chirurgical (20) jusqu'à ce que le couple appliqué à l'aiguille chirurgicale ophtalmique soit dans une plage de couple souhaitée.

9. Procédé selon la revendication 8, comprenant également la notification d'un utilisateur lorsque le couple appliqué à l'aiguille chirurgicale ophtalmique est dans la plage de couple souhaitée par un indicateur visuel et/ou auditif (22) inclus dans le système de chirurgie ophtalmique.

10. Procédé selon la revendication 8, comprenant également la notification d'un utilisateur lorsqu'un remplacement d'un insert de clé amovible (24) est nécessaire.
